# EUROPEAN PATENT SPECIFICATION

(11) **EP 4 188 305 B1**
(45) Date of publication and mention of the grant of the patent: **16.09.2026**
(21) Application number: 21848140.6
(22) Date of filing: 01.02.2021
(51) Int. Cl.: A61G 5/10, A61G 7/02, A61G 9/00, A61F 5/44, A61G 7/015, A61F 5/451

(54) **SYSTEM FOR COLLECTING BODY WASTE OF NON-AMBULATORY PATIENTS**
SYSTEM ZUM SAMMELN VON KÖRPERAUSSCHEIDUNGEN VON NICHT AMBULANTEN PATIENTEN
SYSTÈME DE COLLECTE DE DÉCHETS CORPORELS DE PATIENTS NON AMBULATOIRES

(30) Priority: 30.07.2020 US 202016947393
(43) Date of publication of application: 07.06.2023
(73) Proprietor: Liverpool, Thelma, Rowena, Bronx, NY 10471 (US)
(72) Inventor: Liverpool, Thelma, Rowena, Bronx, NY 10471 (US)
(74) Representative: Jeck, Jonathan
(86) International application number: PCT/US2021/016093
(87) International publication number: WO 2022/025983

(56) References cited:
- CN-A- 107 187 132
- GB-A- 2 322 079
- KR-A- 20040 031 557
- US-A- 4 631 762
- US-A- 4 754 508
- US-A- 4 821 348
- US-A1- 2003 181 880
- US-A1- 2010 094 233
- US-A1- 2010 319 129
- US-A1- 2011 179 571
- US-A1- 2017 020 711
- US-B2- 6 725 485

## Description

### CROSS-REFERENCE TO RELATED APPLICATION

The present PCT patent application claims priority benefit of the U.S. Utility patent application number 16/947,393 and entitled "SYSTEM FOR COLLECTING BODY WASTE OF NON-AMBULATORY PATIENTS", filed on 30-JULY-2020 under 35 USC 111 (a).

From US 2011/0179571 A1, upon which the preamble of claim 1 is based, it is known a therapeutic medical toilet bed having an integrally formed center base mattress supported by a mattress frame adaptable to standard medical bed frames. The bed features a remote-controlled hydraulic retraction system enabling the mattress to assume various positions for patient care and comfort. A functional toilet system is incorporated, wherein the center base mattress retracts to provide toilet access. The toilet system further comprises a pressurized water system and sanitizing unit to cleanse, sanitize, and flush waste downwardly into a high-capacity storage container with additional sanitization capability.

From US 2010/0319129 A1 it is known a negative pressure care bed comprising at least a worktable, an armrest, and a rocking bed having an upper and a lower bed surface, supported within a groove formed by the worktable and armrest via multiple bearing supports. The bed includes a hinged body with upper and lower parts and a first lifter disposed on the upper part. Multiple excretion holes are arranged in both the mattress and the lower bed surface of the rocking bed. A pair of trousers is positioned at the excretion hole, and a bed pan is located within the excretion hole and above the trousers. The bed further includes a waste container with an inlet and an outlet, and a noise reduction oven.

From US 4,631,762 it is known a hospital bed provided with a horizontally shiftable side portion of the mattress, allowing lateral displacement outwardly from the bed frame. The shift creates a central opening between the moved mattress portion and the opposite side, aligned with a vertically adjustable waste receiver positioned beneath the mattress. The receiver moves between a lower position under the mattress and a raised position projecting upwardly into the opening. The receiver includes a lower outlet connected either to a domestic sewer system or a holding tank, and its upper portion incorporates a circumferential manifold designed to discharge fluid downwardly along the receiver's inner walls. Water is supplied to the manifold either from an elevated tank attached to the bed or directly from a domestic pressurized water source.

From GB 2 322 079 A it is known a urine bag assembly comprising a flexible bag and a rigid meter having a urine inlet. The meter is attached to the bag and includes an outlet connected to an inlet of the bag. The meter is pivotally coupled to the bag, allowing it to tip about an axis perpendicular to the plane of the bag to empty its contents into the bag.

From KR 10-2004-0031557 it is known a patient bed designed for convenient defecation for bedridden patients or individuals with limited mobility, as well as for ease of caretaking. The bed comprises conventional vertical legs at each corner and a mattress placed atop. Centrally within the mattress is a vertically penetrating excretion opening. Below this opening, a mounting structure fixed to the legs supports an open-top case positioned at a set distance beneath the opening. The case internally includes front and rear rails along which a front-back moving body is driven via a front-back transfer unit. This moving body supports two vertically movable units-a toilet receptacle and a sealing container-each actuated individually by front and rear lifting units. The toilet receptacle rises into the excretion opening during defecation, whereas the sealing container is inserted to close the opening during normal use. The bed further includes a controller to operate both the front-back transfer unit and the vertical lifting units.

### BACKGROUND OF THE INVENTION

The present invention relates to medical devices and, more particularly, a modification to beds in such a way so they are adapted to collect body waste from patients. The present invention embodies an attachment for modifying beds for collecting feces therefrom, thereby facilitating caring and cleaning for non-ambulatory patients.

To clean fecal matter of non-ambulatory patients, caregivers are required to lift the patient, which is problematic for the following reasons. First, such physical lifting can be hazardous to both parties: for example, back injuries to the caregiver. Also, the use of hoists is common and known to injure both the patient and attendant. Furthermore, among non ambulatory patients an epidemic of obesity is well established, and so operation of hoists in lifting patients usually requires two caretakers. The patient is then undressed and bathed and the procedure reverses. In short, the overall process and the constituent parts of cleaning a non-ambulatory patient takes of the time and effort of the caregiver that could have been directed toward the patient in other endeavors. Moreover, hazards to caregivers and medical personnel from handling infected waste is well known, from Ebola and other diseases.

Non-ambulatory patients develop bedsores and skin infections when improperly or insufficiently cleaned. For the elderly, in particular, these conditions can be life threatening.

Documented instances of neglect and abuse of bedridden patients almost always include the cessation of the changing of soiled undergarments and related cleaning of have been directed toward the patient in other endeavors.

Moreover, hazards to caregivers and medical personnel from handling infected waste is well known, from Ebola and other diseases.

Non-ambulatory patients develop bedsores and skin infections when improperly or insufficiently cleaned. For the elderly, in particular, these conditions can be life threatening.

Documented instances of neglect and abuse of bedridden patients almost always include the cessation of the changing of soiled undergarments and related cleaning of the non-ambulatory patient. And arguably this grueling, thankless task can play a role in the onset of said neglect and abuse. Also noteworthy, the number of adult diapers deposited to landfills is steadily increasing with an aging population.
The environmental issues include groundwater contamination, as well as effects on birds and small animals who forage in these locations.

As can be seen, there is a need for a modification to beds in such a way that they are adapted to collect body waste from non-ambulatory patients. The modification enables the patient to be simply guided into a release position so that body waste is collected without physical touching of the patient - thereafter the patient is easily cleaned up and the overall experience is less traumatic for the patient.

As a result, the use of adult diapers is reduced, the patient will be more comfortable during the bowel movement process, there is minimum effort required of the caregiver except for cleanup, and the patient can also be washed or showered in place instead of being physically moved to a new location - which will reduce stress on the patient and the caregiver.

### SUMMARY OF THE INVENTION

In one aspect of the present invention, a body waste collection system with a mattress is defined in claim 1. The sample port also allows access to the waste for testing while providing an access point for reagents or sensors.

The funnel merges seamlessly with the surface of the bed for normal use.

In yet another aspect of the present invention a method of collecting a body waste from a bedridden patient is defined in claim 5, wherein the method includes the following: providing the above-mentioned body waste collection system; with a mattress positioning the bedridden patient along the non-permeable bedsheet in a release position defined by the waste entry port; and after collecting the body waste lifting distal edges of the bedsheet to assist a flow of the body waste through the waste entry port. This arrangement also allows for washing of the patient.

These and other features, aspects and advantages of the present invention will become better understood with reference to the following drawings, description and claims.

### BRIEF DESCRIPTION OF THE DRAWINGS

Figure 1 is a perspective view of an exemplary embodiment of the present invention, shown in an installed condition;
Figure 2 is a perspective view of an exemplary embodiment of the present invention;
Figure 3 is a side elevation view of an exemplary embodiment of the present invention;
Figure 4 is an exploded perspective view of an exemplary embodiment of the present invention;
Figure 5 is a section view of an exemplary embodiment of the present invention, taken along line 5-5 of Figure 1;
Figure 6 is a detailed view of a plugged waste entry port of an exemplary embodiment of the present invention;
Figure 7 is a side elevation view of an exemplary embodiment of the present invention;
Figure 8 is a side elevation view of an exemplary embodiment of the present invention, illustrating manipulation of sheet 42 to create flow into a waste entry port 34 or 58;
Figure 9 is a detail section view of an exemplary embodiment of the present invention, taken along line 9-9 of Figure 8;
Figure 10 is a perspective view of an exemplary embodiment of the present invention; and
Figure 11 is a perspective view of a wheelchair.

### DETAILED DESCRIPTION OF THE INVENTION

The following detailed description is of the best currently contemplated modes of carrying out exemplary embodiments of the invention. The description is not to be taken in a limiting sense, but is made merely for the purpose of illustrating the general principles of the invention, since the scope of the invention is best defined by the appended claims.

Broadly, an embodiment of the present invention provides a body waste collection system for bedridden or chair-bound patients. A waste entry port may interface with a resting surface of the bed by way of a funnel that terminates at the resting surface. A collection unit may be fluidly coupled to the waste entry port by a flexible conduit system. The collection unit may include a temperature gauge, a volume measure, a sample port, and an air displacement port. Once the bedridden or chair-bound patient has assumed along the resting surface a release position, defined by the waste entry port, no other lifting or movement of the patient is needed to collect their body waste in the collection unit, which can utilize the integrated hardware. At this point, samples of the waste can be removed via the sample port, or the entire collection unit can be removed by disengaging from the flexible unit. Reagents can be added directly to the container in order to give a desired reaction product to aid in analysis of the waste. The researcher is spared physical contact and possible infection from the waste. The waste is spared further contamination by being transferred to other containers.

The container gives visual information such as color, quantity, temperature, physical form [fluid or solid] which may be important to a caregiver. Flow rates can be easily calculated. The presence of sensors in the container may provide information which is transmitted electronically to a remote location or device. These are all parameters which assist in analyzing the state of health of a patient.

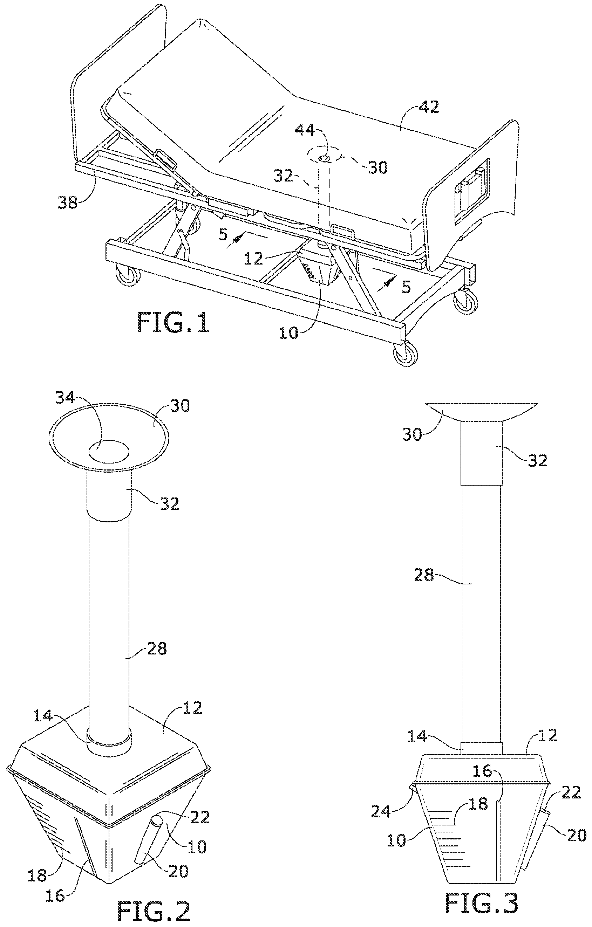

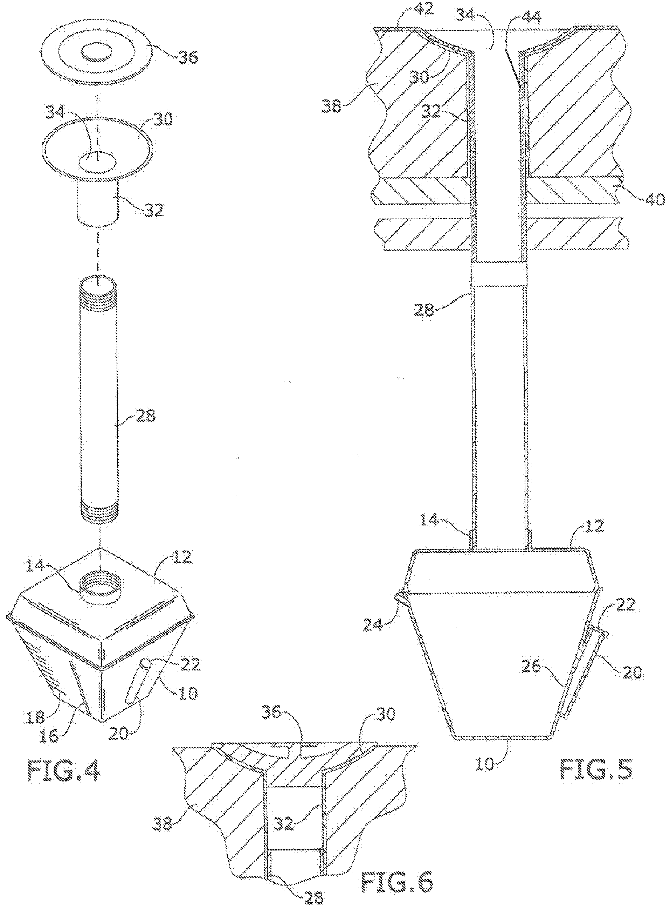

Referring now to Figures 1 through 10, the present invention may include a waste collection unit 10 that is fluidly connectable, by way of a flexible conduit system 28, 32, 60, 70, to a waste entry port 34 or 58 in a resting surface, such as the mattress 38 or 52 of a bed 62.

In certain embodiments, the flexible conduit system 60 may be oriented at transverse directions relative to the solid unit 56. A funnel 30 or 54 may extend upwardly from the waste entry port 34 or 58. A distal end of the funnel 30 or 54 may interface with the resting surface at a drain opening 44. The drain opening 44 may include an opening in a mattress 38, 52 and bed sheet 42 of the bed 62 or the seat 68 of the chair 66. Said interface may define a release position for anatomy of a patient discharging the body waste of fecal matter or urine. The waste entry port 34 or 58 may be closed off by a plug 36.

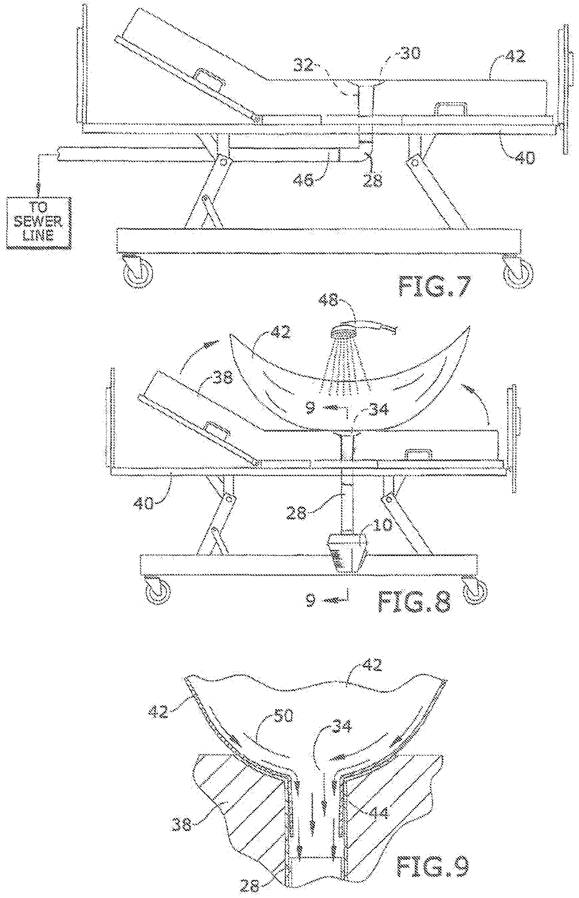

In additional to the funnel 30 or 54 pipe 32, the solid portion of the conduit system will pass through mattress 38, 52. In certain embodiments, the impermeable bedsheet 42 on the mattress 38, 52 - having its own drain opening 44 to interface with the funnel 30 and thus the waste entry port 34 or 58 - can be utilized as a makeshift funnel through lifting up the edges thereof, as illustrated in FIG. 8. In this embodiment, a water source 48 may be used to clean the impermeable bedsheet 42 and flush residual body waste down the waste entry port 24 by way of a flow 50.

The collection unit 10 has as its main opening the flange 14 coupling to the flexible conduit 28 or 70. The volume enclosed by the collection unit 10 is defined by sidewalls. The sidewalls may include a temperature gauge 16 and a volume measure 18. The sidewalls may also provide a sample port 20 with a sample port cap 22 for sealing the sample port 20. The sidewall may also house an air removal port 24.

In certain embodiments, the collection unit 10 can be removed and a sewer conduit 46 may fluidly connect to the flexible conduit 28.

In certain embodiments, a support pillow 64 may facilitate the patient assuming or being guided to the release position, similar to an adjustable hospital bed being moved to likewise assist the patient in assuming the release position. This would also occur when a normal bed is equipped with a portable version of the invention.

A method of using the present invention may include the following. The collection unit 10 disclosed above may be provided. The collection unit 10 may be fluidly coupled to the flexible conduit system 28,and 60, which in turn is fluidly connected to the waste entry port 34 or 58. The patient may be positioned over the drain opening 44 of the overall system disclosed above - at the release position. There the patient can release body waste without having to be moved off their bed.

The collection unit 10 may be removed and the waste diverted to a sewer system - the flexible unit 28 is linked to the sewer line - when the waste is not infectious. The person can be cleaned by paper which is then flushed or a bidet-style cleaning by shower is possible. In certain embodiments, the flexible conduit system can be connected to the home wastewater system when substantial quantities of water are to be removed.

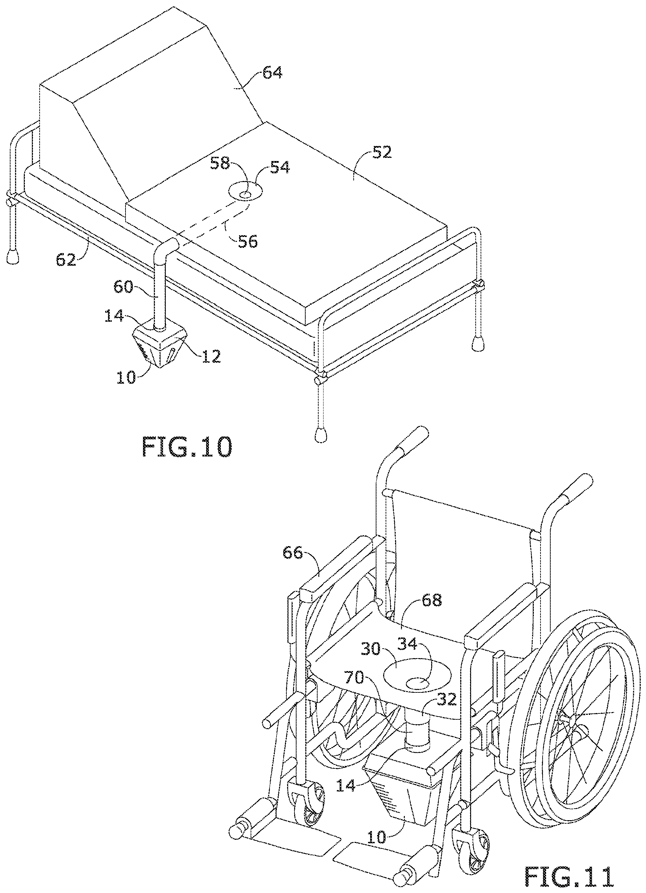

Additionally, the present invention can be made portable, allowing its use in transport vehicles. A portable unit can also be used at home if a bed should not be modified. This is shown in figure 10. The system could also be retrofitted onto wheelchairs per Figure 11, which does not fall under the scope of the invention.

It should be understood, of course, that the foregoing relates to exemplary embodiments of the invention and that modifications may be made without departing from the scope of the invention as set forth in the following claims.

## Claims

1. A body waste collection system for a patient with a mattress (38, 52), comprising:
- a waste entry port (34, 58) interfacing the mattress (38, 52);
- a collection unit (10) fluidly coupled to the waste entry port (34, 58) by way of a flexible conduit system (28, 32, 60,70); and
- a funnel (30, 54) extending upward from the waste entry port (34, 58), wherein the funnel (30, 54) terminates at the mattress (38, 52),
**characterized in that**
- a sample port (20) integrated to the collection unit (10) is provided and
- an impermeable bedsheet (42) is provided on the mattress (38, 52), the bed sheet (42) having its own drain opening (44) interfacing with the funnel 30 and the waste entry port (34, 58) below.

2. The body waste collection system with a mattress of claim 1, wherein a drain opening (44) is formed through the mattress (38, 52).

3. The body waste collection system with a matress of claim 1, further comprising a plug (36) for sealing the waste entry port (34, 58).

4. The body waste collection system with a matress of claim 1, wherein the collection unit includes:
a temperature gauge (16),
a volume measure (18), and
an air displacement port (24).

5. A method of collecting a body waste from a bedridden patient, the method comprising:
- providing the body waste collection system with a matress of claim 3;
- positioning the bedridden patient along said bedsheet (42) in a release position defined by the waste entry port (34, 58); and
- after collecting the body waste lifting distal edges of the bedsheet (42) to assist a flow of the body waste through the waste entry port (34, 58).

## Patentansprüche

1. Körperausscheidungs-Sammelsystem für einen Patienten mit einer Matratze (38, 52), umfassend:
- eine Abfalleintrittsöffnung (34, 58), die mit der Matratze (38, 52) verbunden ist;
- eine Sammeleinheit (10), die über ein
flexibles Leitungssystem (28, 32, 60, 70) fluidisch mit der Abfalleintrittsöffnung (34, 58) gekoppelt ist; und
- einen Trichter (30, 54), der sich von der Abfalleintrittsöffnung (34, 58) nach oben erstreckt, wobei
der Trichter (30, 54) an der Matratze (38, 52) endet,
**dadurch gekennzeichnet, dass**
- eine in die Sammeleinheit (10) integrierte Probenentnahmeöffnung (20) vorgesehen ist und
- ein undurchlässiges Bettlaken (42) auf der Matratze (38, 52) vorgesehen ist, wobei das Bettlaken (42) eine eigene Ablauföffnung (44) aufweist, die mit dem Trichter 30 und
der darunter befindlichen Abfalleintrittsöffnung (34, 58) verbunden ist.

2. Körperausscheidungs-Sammelsystem mit einer Matratze nach Anspruch 1, wobei eine Ablauföffnung (44)
durch die Matratze (38, 52) gebildet ist.

3. Körperausscheidungs-Sammelsystem mit einer Matratze nach Anspruch 1, ferner umfassend einen Stopfen (36) zum
Abdichten der Abfalleintrittsöffnung (34, 58).

4. Körperausscheidungs-Sammelsystem mit einer Matratze nach Anspruch 1, wobei die Sammeleinheit umfasst:
ein Temperaturmessgerät (16),
eine Volumenmesseinrichtung (18), und
eine Luftverdrängungsöffnung (24).

5. Verfahren zum Sammeln von Körperausscheidungen eines bettlägerigen Patienten, wobei das Verfahren
umfasst:
- Bereitstellen des Körperausscheidungs-Sammelsystems mit einer Matratze nach Anspruch 3;
- Positionieren des bettlägerigen Patienten entlang des Bettlakens (42) in einer Abgabeposition,
die durch die Abfalleintrittsöffnung (34, 58) definiert ist; und
- nach dem Sammeln der Körperausscheidungen Anheben distaler Kanten des Bettlakens (42), um
einen Fluss der Körperausscheidungen durch die Abfalleintrittsöffnung (34, 58) zu unterstützen.

## Revendications

1. Système de collecte de déchets corporels pour un patient avec un matelas (38, 52), comprenant :
- un orifice d'entrée de déchets (34, 58) s'interfaçant avec le matelas (38, 52) ;
- une unité de collecte (10) couplée de manière fluidique à l'orifice d'entrée de déchets (34, 58) par l'intermédiaire d'un
système de conduit flexible (28, 32, 60, 70) ; et
- un entonnoir (30, 54) s'étendant vers le haut depuis l'orifice d'entrée de déchets (34, 58), dans lequel
l'entonnoir (30, 54) se termine au niveau du matelas (38, 52),
**caractérisé en ce que**
- un orifice de prélèvement (20) intégré à l'unité de collecte (10) est prévu et
- un drap de lit imperméable (42) est prévu sur le matelas (38, 52), le drap
de lit (42) ayant sa propre ouverture de drainage (44) s'interfaçant avec l'entonnoir 30 et
l'orifice d'entrée de déchets (34, 58) en dessous.

2. Système de collecte de déchets corporels avec un matelas selon la revendication 1, dans lequel une ouverture de drainage (44) est formée
à travers le matelas (38, 52).

3. Système de collecte de déchets corporels avec un matelas selon la revendication 1, comprenant en outre un bouchon (36) pour
obturer l'orifice d'entrée de déchets (34, 58).

4. Système de collecte de déchets corporels avec un matelas selon la revendication 1, dans lequel l'unité de collecte comprend :
une jauge de température (16),
une mesure de volume (18), et
un orifice de déplacement d'air (24).

5. Procédé de collecte de déchets corporels d'un patient alité, le procédé comprenant :
- la fourniture du système de collecte de déchets corporels avec un matelas selon la revendication 3 ;
- le positionnement du patient alité le long dudit drap de lit (42) dans une position de libération
définie par l'orifice d'entrée de déchets (34, 58) ; et
- après la collecte des déchets corporels, le soulèvement des bords distaux du drap de lit (42) pour faciliter
un écoulement des déchets corporels à travers l'orifice d'entrée de déchets (34, 58).
